# EUROPEAN PATENT APPLICATION

(11) **EP 4 414 349 A1**
(43) Date of publication of application: **14.08.2024**
(21) Application number: 22878202.5
(22) Date of filing: 17.08.2022
(51) Int. Cl.: C07C 17/35, C07C 17/42, C07C 21/20

(54) **METHOD FOR PRODUCING HEXAFLUORO-1,3-BUTADIENE**

(30) Priority: 04.10.2021 JP 2021163491
(71) Applicant: Resonac Corporation, Tokyo 105-7325 (JP)
(72) Inventor: TAKAHASHI Kazuma, Tokyo 105-8518 (JP); SUGAWARA Tomokazu, Tokyo 105-8518 (JP); YOROZUYA Shinichi, Tokyo 105-8518 (JP); OGAWA Kouji, Tokyo 105-8518 (JP); MURAYAMA Yoshimasa, Tokyo 105-8518 (JP); KAGA Kazunari, Tokyo 105-8518 (JP)
(74) Representative: Strehl Schübel-Hopf & Partner
(86) International application number: PCT/JP2022/031101
(87) International publication number: WO 2023/058331

(57) **Abstract**

Provided is a method for producing hexafluoro-1,3-butadiene capable of producing hexafluoro-1,3-butadiene at a high yield. The method for producing hexafluoro-1,3-butadiene includes a reaction step of performing dechlorination in which chlorine atoms are eliminated from 1,2,3,4-tetrachlorohexafluorobutane in a reaction solution containing the 1,2,3,4-tetrachlorohexafluorobutane, zinc, at least one of an antioxidant and a polymerization inhibitor, and an organic solvent to yield hexafluoro-1,3-butadiene.

## Description

### Technical Field

The present invention relates to a method for producing hexafluoro-1,3-butadiene.

### Background Art

Hexafluoro-1,3-butadiene is useful, for example, as an etching gas for semiconductor microfabrication. As the method for producing hexafluoro-1,3-butadiene, various methods have been known. For example, PTL 1 discloses a method of dechlorinating 1,2,3,4-tetrachlorohexafluorobutane in 2-propanol in the presence of zinc. PTL 2 discloses a method of dehalogenating 1,4-diiodoperfluorobutane in tetrahydrofuran in the presence of magnesium.

### Citation List

### Patent Literatures

PTL 1: JP 5005681 B
PTL 2: JP 4684401 B

### Summary of Invention

### Technical Problem

By the methods disclosed in PTLs 1 and 2, however, peroxides may be generated as by-products during the reactions, and radicals arising from the peroxides may cause side reactions. The side reaction may generate impurities or polymers, and this may reduce the yield of hexafluoro-1,3-butadiene.

The present invention is intended to provide a production method capable of producing hexafluoro-1,3-butadiene at a high yield.

### Solution to Problem

To solve the problems, aspects of the present invention are the following [1] to [8].
[1] A method for producing hexafluoro-1,3-butadiene, the method including a reaction step of performing dechlorination in which chlorine atoms are eliminated from 1,2,3,4-tetrachlorohexafluorobutane in a reaction solution containing the 1,2,3,4-tetrachlorohexafluorobutane, zinc, at least one of an antioxidant and a polymerization inhibitor, and an organic solvent to yield hexafluoro-1,3-butadiene.
[2] The method for producing hexafluoro-1,3-butadiene according to the aspect [1], in which the antioxidant is at least one of a radical chain initiation inhibitor, a radical scavenger, and a peroxide decomposer.
[3] The method for producing hexafluoro-1,3-butadiene according to the aspect [1], in which the antioxidant is a peroxide decomposer.
[4] The method for producing hexafluoro-1,3-butadiene according to the aspect [2] or [3], in which the peroxide decomposer is 10H-phenothiazine.
[5] The method for producing hexafluoro-1,3-butadiene according to any one of the aspects [1] to [4], in which the polymerization inhibitor is a compound having a cyclohexadiene ring structure.
[6] The method for producing hexafluoro-1,3-butadiene according to any one of the aspects [1] to [5], in which the ratio of the total molar amount of the antioxidant and the polymerization inhibitor to the molar amount of the 1,2,3,4-tetrachlorohexafluorobutane is 0.01% or more and 10% or less.
[7] The method for producing hexafluoro-1,3-butadiene according to any one of the aspects [1] to [6], in which the organic solvent is an alcohol.
[8] The method for producing hexafluoro-1,3-butadiene according to the aspect [7], in which the alcohol is at least one of methanol, ethanol, 1-propanol, 2-propanol, 1-butanol, and 2-butanol.

### Advantageous Effects of Invention

According to the present invention, hexafluoro-1,3-butadiene can be produced at a high yield.

### Brief Description of Drawings

FIG. 1 is a view illustrating a reaction pathway in an embodiment of the method for producing hexafluoro-1,3-butadiene pertaining to the present invention; and
FIG. 2 is a schematic view illustrating the structure of a production apparatus of hexafluoro-1,3-butadiene used in Examples and Comparative Examples.

### Description of Embodiments

Embodiments of the present invention will now be described. The embodiments are merely examples of the present invention, and the present invention is not limited to the embodiments. Various modifications or improvements can be made in the embodiments, and such modifications and improvements can be encompassed by the present invention.

In a conventional method for producing hexafluoro-1,3-butadiene, a reaction of eliminating all the chlorine atoms from 1,2,3,4-tetrachlorohexafluorobutane is performed in a reaction solution containing 1,2,3,4-tetrachlorohexafluorobutane, zinc, and an organic solvent to yield hexafluoro-1,3-butadiene.

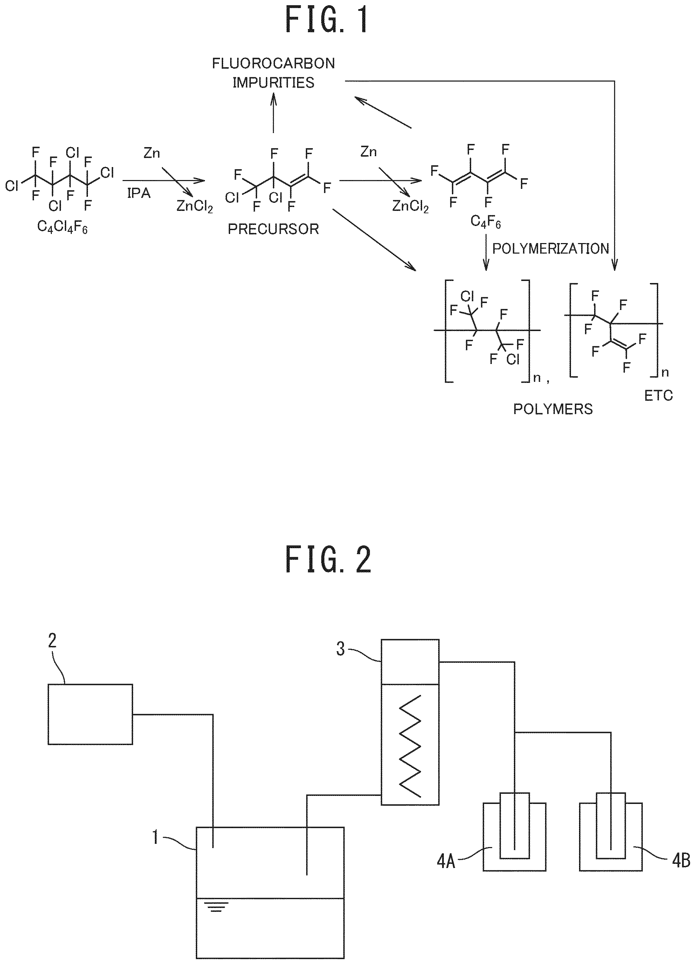

In the above conventional method for producing hexafluoro-1,3-butadiene, why the yield of hexafluoro-1,3-butadiene is reduced has not been completely revealed, but the inventors of the present invention have found one reason of the yield reduction as follows: peroxides generated as by-products during the reaction cause side reactions; and the side reactions yield impurities or polymers. The reaction pathway when 1,2,3,4-tetrachlorohexafluorobutane (C₄Cl₄F₆) is used as a material to produce hexafluoro-1,3-butadiene (C₄F₆) will be described with reference to FIG. 1.

When 1,2,3,4-tetrachlorohexafluorobutane is subjected to dechlorination in an organic solvent in the presence of zinc (Zn), the first dechlorinating yields 3,4-dichloro-1,1,2,3,4,4-hexafluoro-1-butene (C₄Cl₂F₆) as a precursor of hexafluoro-1,3-butadiene, and subsequently dechlorinating the 3,4-dichloro-1,1,2,3,4,4-hexafluoro-1-butene (second dechlorinating) yields hexafluoro-1,3-butadiene. Hereinafter, 3,4-dichloro-1,1,2,3,4,4-hexafluoro-1-butene is also called a "3,4-dichloro product".

In the dechlorination as described above, why fluorocarbon impurities or polymers are generated as by-products has not been revealed yet, but the 3,4-dichloro product as the precursor and hexafluoro-1,3-butadiene as the target compound probably undergo side reactions to yield fluorocarbon impurities or polymers, as shown in FIG. 1.

The inventors of the present invention have therefore conducted intensive studies and consequently have found that by performing the dechlorination in the presence of at least one of an antioxidant and a polymerization inhibitor, the generation of fluorocarbon impurities and polymers as by-products is suppressed, and hexafluoro-1,3-butadiene is produced at a higher yield. Why the presence of an antioxidant or a polymerization inhibitor suppresses the generation of fluorocarbon impurities and polymers as by-products is not clear, but the reason is probably as follows: peroxides generated as by-products during the reaction are reduced by the antioxidant or the polymerization inhibitor; this suppresses side reactions caused by radicals arising from the peroxides to prevent the generation of fluorocarbon impurities or polymer as by-products; and hexafluoro-1,3-butadiene is produced at a higher yield.

In other words, the method for producing hexafluoro-1,3-butadiene pertaining to the present embodiment includes a reaction step of performing dechlorination in which chlorine atoms are eliminated from 1,2,3,4-tetrachlorohexafluorobutane in a reaction solution containing the 1,2,3,4-tetrachlorohexafluorobutane, zinc, at least one of an antioxidant and a polymerization inhibitor, and an organic solvent to yield hexafluoro-1,3-butadiene.

By the method for producing hexafluoro-1,3-butadiene pertaining to the present embodiment, at least one of an antioxidant and a polymerization inhibitor suppresses the generation of fluorocarbon impurities or polymers as by-products, and accordingly hexafluoro-1,3-butadiene can be produced at a high yield (for example, a yield of 85% or more).

The amount of hexafluoro-1,3-butadiene as the main product and the amount of fluorocarbon impurities as by-products can be determined by analysis using gas chromatography. The amount of polymers can be estimated by subtracting the quantified amount of hexafluoro-1,3-butadiene and the quantified amount of the fluorocarbon impurities from the used amount of 1,2,3,4-tetrachlorohexafluorobutane.

Fluorocarbon impurities and polymers will next be described. The fluorocarbon impurities are exemplified by compounds represented by chemical formula C₄HₓCl_{y}F_{z} (x, y, and z are each a positive integer). Examples include pentafluoro-1,3-butadiene (C₄HF₅), chloropentafluoro-1,3-butadiene (C₄ClF₅), tetrafluoro-1,3-butadiene (C₄H₂F₄), and dichlorohexafluorobutene (C₄Cl₂F₆ (except the 3,4-dichloro product as the precursor)). These fluorocarbon impurities have a carbon-carbon double bond and thus are polymerizable.

The fluorocarbon impurities are also exemplified by compounds represented by chemical formula C₄HₐO_{b}Cl_{c}F_{d} (a, b, c, and d are each a positive integer). Examples include C₄OF₆, C₄OCl₂F₆, and polymers such as a dimer and a trimer of them.

Examples of the polymer include a polymer of hexafluoro-1,3-butadiene, a polymer of the 3,4-dichloro product as the precursor, a copolymer of hexafluoro-1,3-butadiene and the 3,4-dichloro product as the precursor, and a polymer of fluorocarbon impurities (see FIG. 1).

In the present invention, "hexafluoro-1,3-butadiene" means "1,1,2,3,4,4-hexafluoro-1,3-butadiene", and "1,2,3,4-tetrachlorohexafluorobutane" means "1,2,3,4-tetrachloro-1,1,2,3,4,4-hexafluorobutane".

Hereinafter, the method for producing hexafluoro-1,3-butadiene pertaining to the present embodiment will be described in further detail.

### [Antioxidant and polymerization inhibitor]

The antioxidant may be any type that is unlikely to inhibit dechlorination of eliminating chlorine atoms from 1,2,3,4-tetrachlorohexafluorobutane to form hexafluoro-1,3-butadiene (hereinafter also simply called "dechlorination"), has poor reactivity with zinc, and is soluble in an organic solvent. Examples include a radical chain initiation inhibitor, a radical scavenger, and a peroxide decomposer.

The radical chain initiation inhibitor includes hydrazide antioxidants and amide antioxidants. Specific examples of the radical chain initiation inhibitor include N-salicyloyl-N'-aldehydehydrazine, N-salicyloyl-N'-acetylhydrazine, N,N'-diphenyloxamide, and N,N'-di(2-hydroxyphenyl) oxamide.

The radical scavenger includes phenol antioxidants and amine antioxidants. Specific examples of the radical scavenger include 3,5-di-tert-butyl-4-hydroxytoluene (C₁₅H₂₄O), 2,2'-methylenebis (4-methyl-6-tert-butylphenol) (C₂₃H₃₂NO₂), 4-hydroxy-2,2, 6, 6-tetramethylpiperidine 1-oxyl free radical (C₉H₁₈NO₂), and N,N'-di-2-naphthyl-1,4-phenylenediamine (C₂₆H₂₀N₂).

The peroxide decomposer includes sulfur-containing antioxidants and phosphorus-containing antioxidants. Specific examples of the peroxide decomposer include 10H-phenothiazine (C₁₂H₉NS), methyl-10H-phenothiazine (C₁₃H₁₁NS), chloro-10H-phenothiazine (C₁₂H₈ClNS), fluoro-10H-phenothiazine (C₁₂H₈FNS), 2-mercaptobenzimidazole (C₇H₆N₂S), terpene sulfide, triisodecyl phosphite (C₃₀H₆₃O₃P), and triphenyl phosphite (C₁₈H₁₅O₃P).

The polymerization inhibitor may be any type, and a compound having a cyclohexadiene ring structure may be used. Specific examples of the compound having a cyclohexadiene ring structure include α-terpinene (4-methyl-1-(1-methylethyl)-1,3-cyclohexadiene (C₁₀H₁₆)), γ-terpinene (4-methyl-1-(1-methylethyl)-1,4-cyclohexadiene (C₁₀H₁₆)), and α-terpinolene (1-methyl-4-isopropylidene-1-cyclohexene (C₁₀H₁₆)).

These antioxidants and polymerization inhibitors may be used singly or in combination of two or more of them.

Of these antioxidants and polymerization inhibitors, 10H-phenothiazine, which effectively increases the yield of hexafluoro-1,3-butadiene, is most preferred.

10H-Phenothiazine has a boiling point of 371°C, and hexafluoro-1,3-butadiene has a boiling point of 5.4°C. When the reaction temperature of dechlorination is, for example, set at 50 to 100°C, the vapor formed by vaporization of the reaction solution mainly contains hexafluoro-1,3-butadiene and an organic solvent, and 10H-phenothiazine is probably contained in a trace amount in the vapor.

If a vapor formed by vaporization of a reaction solution contains 10H-phenothiazine, 10H-phenothiazine can be easily separated from the vapor through a purification process by distillation or adsorption. Hence, 10H-phenothiazine can be used in the production process of hexafluoro-1,3-butadiene.

When the antioxidant and the polymerization inhibitor are used in a larger total amount (when the antioxidant and the polymerization inhibitor are contained at a higher total concentration in a reaction solution), hexafluoro-1,3-butadiene is likely to be produced at a higher yield, but the ratio of the total molar amount of the antioxidant and the polymerization inhibitor to the molar amount of 1,2,3,4-tetrachlorohexafluorobutane is preferably 0.01% or more and 10% or less, more preferably 0.05% or more and 5% or less, and even more preferably 0.1% or more and 2% or less.

The antioxidant and the polymerization inhibitor may be dissolved in at least one of an organic solvent and 1,2,3,4-tetrachlorohexafluorobutane, and the solution may be fed to a reaction container such as an autoclave for dechlorination. Alternatively, the antioxidant and the polymerization inhibitor may be directly fed to a reaction container for dechlorination.

When the antioxidant and the polymerization inhibitor are directly fed to a batch-type reaction container, the order of feeding the antioxidant, the polymerization inhibitor, zinc, and an organic solvent to the reaction container is not specifically limited, and these materials may be fed in any order. For example, to a reaction container containing zinc and an organic solvent, the antioxidant and the polymerization inhibitor may be fed. Alternatively, to a reaction container containing the antioxidant and the polymerization inhibitor, zinc and an organic solvent may be fed. When the reaction step is performed in a continuous-type reaction container, the antioxidant, the polymerization inhibitor, zinc, and an organic solvent are required to be fed to the reaction container at the same time.

### [Zinc]

Zinc may be in any form as long as the dechlorination proceeds, but is preferably in a powder form from the viewpoint of reactivity or handling properties. The powder zinc preferably has an average particle size of 0.04 mm or more and 1.0 mm or less, more preferably 0.04 mm or more and 0.50 mm or less, and even more preferably 0.04 mm or more and 0.10 mm or less.

Zinc may be used in any amount as long as dechlorination proceeds, but the ratio of the molar amount of zinc to the molar amount of 1,2,3,4-tetrachlorohexafluorobutane (zinc/1,2,3,4-tetrachlorohexafluorobutane) is preferably 0.1 or more and 10 or less, more preferably 1.0 or more and 5.0 or less, and even more preferably 2.0 or more and 3.0 or less.

### [Organic solvent]

The organic solvent may be any type that does not inhibit dechlorination. Preferably, the organic solvent easily dissolves an antioxidant, has poor reactivity with zinc, well disperses zinc, and has non-zero solubility of zinc chloride (ZnCl₂) as a by-product.

Examples of the suitably usable organic solvent include alcohols, cyclic ethers, acetone (C₂H₆CO), acetonitrile (CH₃CN), aromatic hydrocarbons, amide solvents, organic acids, N-methyl-2-pyrrolidone (C₅H₉NO), and mixed solvents of them. Of these organic solvents, an alcohol allows dechlorination to suitably proceed and thus is preferred.

Specific examples of the alcohol include methanol (CH₃OH), ethanol (C₂H₅OH), 1-propanol (C₃H₇OH), 2-propanol (C₃H₇OH), 1-butanol (C₄H₉OH), and 2-butanol (C₄H₉OH). Of them, 2-propanol is most preferred from the viewpoint of handling properties.

Examples of the cyclic ether include tetrahydrofuran (C₄H₈O) and 1,4-dioxane (C₄H₈O₂). Examples of the aromatic hydrocarbon include benzene (C₆H₆) and toluene (C₇H₈). Examples of the amide solvent include N,N-dimethylformamide (C₃H₇NO). Examples of the organic acid include acetic acid (CH₃COOH). Organic solvents may be used singly or in combination of two or more of them.

The organic solvent may be used in any amount as long as dechlorination proceeds, but the ratio of the molar amount of the organic solvent to the molar amount of 1,2,3,4-tetrachlorohexafluorobutane (organic solvent/1,2,3,4-tetrachlorohexafluorobutane) is preferably 0.1 or more and 10 or less, more preferably 1.0 or more and 9.0 or less, and even more preferably 3.0 or more and 8.0 or less.

### [Reaction conditions]

The reaction temperature of dechlorination is not specifically limited as long as dechlorination proceeds, but is preferably 20°C or more and 150°C or less, more preferably 40°C or more and 130°C or less, and even more preferably 70°C or more and 100°C or less.

The reaction pressure of dechlorination is not specifically limited as long as dechlorination proceeds, but is preferably 0.01 MPa or more and 1 MPa or less in terms of absolute pressure, more preferably 0.05 MPa or more and 0.5 MPa or less in terms of absolute pressure, and even more preferably 0.08 MPa or more and 0.2 MPa or less in terms of absolute pressure.

### Examples

The present invention will next be described in further detail with reference to Examples and Comparative Examples.

### [Example 1]

A production apparatus of hexafluoro-1,3-butadiene illustrated in FIG. 2 was used to perform reaction. In an SUS316 autoclave 1 having an internal volume of 1 L, 471 g (7.84 mol) of 2-propanol as an organic solvent and 0.39 g (0.0020 mol) of 10H-phenothiazine (in Table 1, denoted by "PTZ") as the antioxidant were placed, and 10H-phenothiazine was dissolved in 2-propanol. To the solution, 327 g of metal zinc powder (5.00 mol; an average particle size of 0.075 mm; the particle size distribution was determined by using a laser diffraction measuring system, Microtrac MT3300 manufactured by MicrotracBEL; the average particle size was calculated from an average area; the number of measurement n was three) was added. The ratio of the used amount (molar amount) of 10H-phenothiazine to the used amount (molar amount) of 1,2,3,4-tetrachlorohexafluorobutane was 0.1%. The autoclave 1 was equipped with a jacket (not illustrated) having a heating structure and a stirrer (not illustrated) at the upper part, and the heating system was a jacket heating system.

While the contents in the autoclave 1 were stirred, the temperature was increased to 80°C. To the outlet of the autoclave 1, a Dimroth condenser 3 was attached. While the temperature of the contents in the autoclave 1 was maintained at 80°C under normal pressure, 608 g (2.00 mol) of 1,2,3,4-tetrachlorohexafluorobutane was added dropwise at a speed of 2.0 g per min from a feeder 2 for 1,2,3,4-tetrachlorohexafluorobutane, and reaction was performed.

After dropwise addition over about 5 hours, the temperature of the contents was increased to 95°C, and a part of 2-propanol and the product were vaporized at normal pressure over 2 hours. The vapor was fed to a trap (first trap 4A) cooled at -78°C to be liquified, and 263.2 g of a first fraction was collected. The first fraction was analyzed by gas chromatography to be a crude hexafluoro-1,3-butadiene containing hexafluoro-1,3-butadiene at a content of 95.3% by mass.

Next, the temperature of the contents in the autoclave 1 was returned to normal temperature, and the operation of the Dimroth condenser 3 was stopped. Through the autoclave 1, nitrogen gas (N₂) was then allowed to flow at a flow rate of 100 mL/min, and the products remaining in the autoclave 1 were vaporized. The vapor was fed to a trap (second trap 4B) cooled at - 78°C to be liquified, and 8.0 g of a second fraction was collected. The second fraction was analyzed by gas chromatography to be a crude hexafluoro-1,3-butadiene containing hexafluoro-1,3-butadiene at a content of 84.1% by mass. The yield of hexafluoro-1,3-butadiene in the first fraction and the second fraction in total (in Table 1, denoted by "C₄F₆ in fractions") was 82.8%. The components not vaporized but remaining in the autoclave 1 (including the solvent, polymers, and by-product impurities) were analyzed as the residue.

The yield of C₄F₆ was defined in the following manner. Yield of C4F6 (%) = {[mass of first fraction] × [content of hexafluoro-1,3-butadiene in first fraction] / [molecular weight of C4F6 (162.03)] + [mass of second fraction] × [content of hexafluoro-1,3-butadiene in second fraction] / [molecular weight of C4F6 (162.03)]} / [molar number of used 1,2,3,4-tetrachlorohexafluorobutane] × 100

The residue remaining in the autoclave 1 after collection of the first fraction and the second fraction was analyzed by gas chromatography. From the analysis results of the first fraction, the second fraction, and the residue, the reaction in Example 1 generated C₄Cl₂F₆ (except the precursor), fluorocarbon impurities other than C₄Cl₂F₆, and polymers as by-products.

The ratio (yield) of the molar number of C₄Cl₂F₆ (except the precursor) as by-products to the molar number of used 1,2,3,4-tetrachlorohexafluorobutane was 1.8%, the ratio (yield) of the molar number of fluorocarbon impurities other than C₄Cl₂F₆ as by-products (in Table 1, denoted by "others") to the molar number of used 1,2,3,4-tetrachlorohexafluorobutane was 6.7%, and the ratio (yield) of the molar number of polymers as by-products to the molar number of used 1,2,3,4-tetrachlorohexafluorobutane was 6.6%. The ratio of the molar number of C₄F₆ remaining in the residue to the molar number of used 1,2,3,4-tetrachlorohexafluorobutane was 1.9% (the yield of C₄F₆ in the residue was 1.9%).

The conversion rate of used 1,2,3,4-tetrachlorohexafluorobutane was 100%, and products other than C₄F₆, C₄Cl₂F₆ (except the precursor) and fluorocarbon impurities were considered as polymers. The molar number of polymers as by-products was not calculated from the molecular weight of the polymers but was calculated in a simplified manner: the total molar number of C₄F₆, C₄Cl₂F₆ (except the precursor), and fluorocarbon impurities was subtracted from the molar number of used 1,2,3,4-tetrachlorohexafluorobutane. The above results are summarized in Table 1.

**[Table 1]**

| | Antioxidant/polymerization inhibitor | | Yield of C₄F₆ (%) | Yield of C₄F₆ in residue (%) | Yield of fron impurities (%) | | Yield of polymers (%) |
|---|---|---|---|---|---|---|---|
| | Type | Molar ratio (%) | | | C₄Cl₂F₆ *⁾ | Others | |
| Comp. Ex. 1 | Without | - | 71.7 | 4.6 | 3.8 | 12.2 | 7.7 |
| Ex. 1 | PTZ | 0.1 | 82.8 | 1.9 | 1.8 | 6.9 | 6.6 |
| Ex. 2 | PTZ | 1.0 | 85.0 | 1.0 | 1.3 | 6.8 | 5.9 |
| Ex. 3 | BHT | 1.0 | 73.5 | 5.3 | 3.9 | 10.9 | 6.4 |
| Ex. 4 | α-terpinene | 1.0 | 73.7 | 5.6 | 4.3 | 10.5 | 5.9 |
| Ex. 5 | 4H-TEMPO | 1.0 | 73.9 | 4.2 | 3.4 | 9.7 | 8.8 |

| | | | | | | | |
|---|---|---|---|---|---|---|---|
| *) Other than 3,4-dichloro product | | | | | | | |

The measurement conditions for gas chromatography of the first fraction and the second fraction were as follows;
Measurement apparatus: a gas chromatograph, GC-2014 manufactured by Shimadzu Corporation
Column: a column for gas chromatography, CP-PoraPLOT Q-HT manufactured by Agilent Technologies
Column temperature rise conditions: 90°C (0 min) → (5°C/min) → 230°C (5 min)
Column equilibrium time: 1 min
Initial temperature of inlet: 120°C
Control mode: pressure
Pressure: 46.5 kPa (constant)
Total flow rate: 54.0 mL/min
Column flow rate: 1.0 mL/min
Linear velocity: 23.1 cm/sec
Split ratio: 50
Carrier gas: He
Detector: hydrogen flame ionization detector (FID)
Detector temperature: 230°C
Hydrogen pressure: 60 kPa
Air pressure: 50 kPa
Sample injection volume: 0.2 mL (gas)

Measurement conditions for gas chromatography of the residue were as follows;
Measurement apparatus: a gas chromatograph, GC-2014 manufactured by Shimadzu Corporation
Column: a column for gas chromatography, DB-1 manufactured by Agilent Technologies
Column temperature rise conditions: 40°C (15 min) → (10°C/min) → 230°C (31 min)
Column equilibrium time: 2 min
Initial temperature of inlet: 200°C
Control mode: pressure
Pressure: 100 kPa (constant)
Total flow rate: 24.1 mL/min
Column flow rate: 1.75 mL/min
Linear velocity: 25 cm/sec
Purge flow rate: 3 mL/min
Split ratio: 11
Carrier gas: He
Detector: hydrogen flame ionization detector (FID)
Detector temperature: 230°C
Hydrogen pressure: 60 kPa
Air pressure: 50 kPa
Sample injection volume: 0.2 mL (liquid)

### [Example 2]

Dechlorination was performed in the same manner as in Example 1 except that the amount of used 10H-phenothiazine was 3.9 g (0.020 mol). The ratio of the used amount (molar amount) of 10H-phenothiazine to the used amount (molar amount) of 1,2,3,4-tetrachlorohexafluorobutane was 1.0%. As a result, 283.0 g of a first fraction, 7.8 g of a second fraction, and a residue were yielded.

The first fraction, the second fraction, and the residue were analyzed by gas chromatography. The first fraction contained hexafluoro-1,3-butadiene at a content of 95.4% by mass, and the second fraction contained hexafluoro-1,3-butadiene at a content of 86.3% by mass. The yield of hexafluoro-1,3-butadiene in the first fraction and the second fraction in total was 85.0%. The results analyzed in the same manner as in Example 1 are summarized in Table 1.

### [Example 3]

Dechlorination was performed in the same manner as in Example 1 except that 4.4 g (0.020 mol) of 3,5-di-tert-butyl-4-hydroxytoluene (in Table 1, denoted by "BHT") was used as the antioxidant in place of 10H-phenothiazine. The ratio of the used amount (molar amount) of 3,5-di-tert-butyl-4-hydroxytoluene to the used amount (molar amount) of 1,2,3,4-tetrachlorohexafluorobutane was 1.0%. As a result, 223.9 g of a first fraction, 32.0 g of a second fraction, and a residue were yielded.

The first fraction, the second fraction, and the residue were analyzed by gas chromatography. The first fraction contained hexafluoro-1,3-butadiene at a content of 94.5% by mass, and the second fraction contained hexafluoro-1,3-butadiene at a content of 85.3% by mass. The yield of hexafluoro-1,3-butadiene in the first fraction and the second fraction in total was 73.5%. The results analyzed in the same manner as in Example 1 are summarized in Table 1.

### [Example 4]

Dechlorination was performed in the same manner as in Example 1 except that 2.7 g (0.020 mol) of α-terpinene as a polymerization inhibitor was used in place of the antioxidant. The ratio of the used amount (molar amount) of α-terpinene to the used amount (molar amount) of 1,2,3,4-tetrachlorohexafluorobutane was 1.0%. As a result, 208.3 g of a first fraction, 46.8 g of a second fraction, and a residue were yielded.

The first fraction, the second fraction, and the residue were analyzed by gas chromatography. The first fraction contained hexafluoro-1,3-butadiene at a content of 95.1% by mass, and the second fraction contained hexafluoro-1,3-butadiene at a content of 88.6% by mass. The yield of hexafluoro-1,3-butadiene in the first fraction and the second fraction in total was 73.7%. The results analyzed in the same manner as in Example 1 are summarized in Table 1.

### [Example 5]

Dechlorination was performed in the same manner as in Example 1 except that 3.4 g (0.020 mol) of 4-hydroxy-2,2,6,6-tetramethylpiperidine 1-oxyl free radical (in Table 1, denoted by "4H-TEMPO") was used as the antioxidant in place of 10H-phenothiazine. The ratio of the used amount (molar amount) of 4-hydroxy-2,2,6,6-tetramethylpiperidine 1-oxyl free radical to the used amount (molar amount) of 1,2,3,4-tetrachlorohexafluorobutane was 1.0%. As a result, 215.0 g of a first fraction, 40.5 g of a second fraction, and a residue were yielded.

The first fraction, the second fraction, and the residue were analyzed by gas chromatography. The first fraction contained hexafluoro-1,3-butadiene at a content of 94.8% by mass, and the second fraction contained hexafluoro-1,3-butadiene at a content of 88.6% by mass. The yield of hexafluoro-1,3-butadiene in the first fraction and the second fraction in total was 73.9%. The results analyzed in the same manner as in Example 1 are summarized in Table 1.

### [Comparative Example 1]

Dechlorination was performed in the same manner as in Example 1 except that neither the antioxidant nor the polymerization inhibitor was used. As a result, 207.0 g of a first fraction, 42.7 g of a second fraction, and a residue were yielded.

The first fraction, the second fraction, and the residue were analyzed by gas chromatography. The first fraction contained hexafluoro-1,3-butadiene at a content of 94.6% by mass, and the second fraction contained hexafluoro-1,3-butadiene at a content of 87.2% by mass. The yield of hexafluoro-1,3-butadiene in the first fraction and the second fraction in total was 71.7%. The results analyzed in the same manner as in Example 1 are summarized in Table 1.

### Reference Signs List

- 1: autoclave
- 2: feeder
- 3: Dimroth condenser
- 4A: first trap
- 4B: second trap

## Claims

1. A method for producing hexafluoro-1,3-butadiene, the method comprising:
a reaction step of performing dechlorination in which a chlorine atom is eliminated from 1,2,3,4-tetrachlorohexafluorobutane in a reaction solution containing the 1,2,3,4-tetrachlorohexafluorobutane, zinc, at least one of an antioxidant and a polymerization inhibitor, and an organic solvent to yield hexafluoro-1,3-butadiene.

2. The method for producing hexafluoro-1,3-butadiene according to claim 1, wherein the antioxidant is at least one of a radical chain initiation inhibitor, a radical scavenger, and a peroxide decomposer.

3. The method for producing hexafluoro-1,3-butadiene according to claim 1, wherein the antioxidant is a peroxide decomposer.

4. The method for producing hexafluoro-1,3-butadiene according to claim 2 or claim 3, wherein the peroxide decomposer is 10H-phenothiazine.

5. The method for producing hexafluoro-1,3-butadiene according to any one of claims 1 to 4, wherein the polymerization inhibitor is a compound having a cyclohexadiene ring structure.

6. The method for producing hexafluoro-1,3-butadiene according to any one of claims 1 to 5, wherein a ratio of a total molar amount of the antioxidant and the polymerization inhibitor to a molar amount of the 1,2,3,4-tetrachlorohexafluorobutane is 0.01% or more and 10% or less.

7. The method for producing hexafluoro-1,3-butadiene according to any one of claims 1 to 6, wherein the organic solvent is an alcohol.

8. The method for producing hexafluoro-1,3-butadiene according to claim 7, wherein the alcohol is at least one of methanol, ethanol, 1-propanol, 2-propanol, 1-butanol, and 2-butanol.
